(19) Europäisches Patentamt
European Patent Office
Office européen des brevets

(11) EP 0 623 615 B1

(12) # EUROPÄISCHE PATENTSCHRIFT

(45) Veröffentlichungstag und Bekanntmachung des Hinweises auf die Patenterteilung:
**30.06.1999 Patentblatt 1999/26**

(21) Anmeldenummer: **94106049.3**

(22) Anmeldetag: **19.04.1994**

(51) Int Cl.6: **C07D 413/06**, C07D 263/20, A61K 31/42, A61K 31/495, C07D 491/10
// (C07D491/10, 307:00, 221:00)

(54) **Substituierte 1-Phenyl-oxazolidin-2-on Derivate, deren Herstellung und deren Verwendung als Adhäsionsrezeptor-Antagonisten**

Substituted 1-phenyl-oxazolidin-2-one derivatives, their preparation and their use as adhesion-receptor antagonists

Dérivés de 1-phényl-oxazolidin-2-one substitués, leur préparation et leur utilisation comme antagonistes du récepteur d'adhésion

(84) Benannte Vertragsstaaten:
**AT BE CH DE DK ES FR GB GR IE IT LI LU NL PT SE**

(30) Priorität: **01.05.1993 DE 4314378**
**22.02.1994 DE 4405633**

(43) Veröffentlichungstag der Anmeldung:
**09.11.1994 Patentblatt 1994/45**

(73) Patentinhaber: **MERCK PATENT GmbH**
**64293 Darmstadt (DE)**

(72) Erfinder:
- **Raddatz, Peter, Dr.**
  **D-64342 Seeheim-Jugenheim (DE)**
- **Gante, Joachim, Prof.**
  **D-64291 Darmstadt (DE)**
- **Juraszyk, Horst, Dr.**
  **D-63342 Seeheim (DE)**
- **Wurziger, Hanns, Dr.**
  **D-64291 Darmstadt (DE)**
- **Prücher, Helmut**
  **D-64646 Heppenheim (DE)**
- **Bernotat-Danielowski, Sabine, Dr.**
  **D-61231 Bad Naumheim (DE)**
- **Melzer, Guido**
  **D-65719 Hofheim/Ts. (DE)**

(56) Entgegenhaltungen:
**EP-A- 0 300 272**
**EP-A- 0 381 033**
**EP-A- 0 443 197**
**EP-A- 0 635 505**
**DE-A- 1 951 273**

EP 0 623 615 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Verbindungen der Formel I

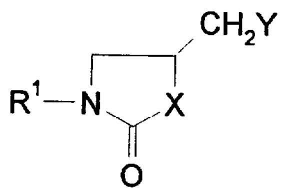

I

worin

X O,

Y einen unsubstituierten oder einen einfach durch $R^2$ substituierten Aziridino-, Azetidino-, Pyrrolidino-, Piperidino-, 1-Oxa-8-azaspiro[4,5]decan-8-yl-, Hexahydroazepino- oder 4-$R^4$-piperazino-rest, der zusätzlich durch eine Gruppe OZ, SZ oder $N(Z)_2$ und/oder durch Carbonylsauerstoff substituiert sein kann,

Z jeweils H, A, Phenyl-$C_kH_{2k}$- oder Acyl mit 1-11 C-Atomen,

$R^1$ einen einfach durch CN, $H_2N$-$CH_2$-, $(A)_2N$-$CH_2$-, $H_2N$-C(=NH)-, $H_2N$-C(=NH)-NH-, $H_2N$-C(=NH)-NH-$CH_2$-, HO-NH-C(=NH)- oder HO-NH-C(=NH)-NH substituierten Phenylrest,

$R^2$ -$C_mH_{2m}$-$COOR^3$ oder -$C_nH_{2n}$-O-$C_pH_{2p}$-$COOR^3$,

$R^3$ H, A oder Benzyl,

$R^4$ H, A, Benzyl oder -$C_mH_{2m}$-$COOR^3$,

A jeweils Alkyl mit 1-6 C-Atomen,

k und m jeweils 0, 1, 2 oder 3,

n 0, 1 oder 2, und

p 1, 2 oder 3 bedeuten,

sowie deren Salze.

Ähnliche Verbindungen sind aus der EP-A1-0 381 033 bekannt.

In der DE 195 12 73 sind 5-Aminomethyloxazolidin-2-on-Derivate beschrieben.

Andere Oxazolidinone mit Wirkungen auf das Zentralnervensystem kennt man z. B. aus der EP 0 300 272 oder aus EP 0 443 197. Verbindungen, die anstelle des Oxazolidin-2-on-Rings eine Amid- oder Sulfonamidruppe aufweisen, sind in der EP 0 381 033 beschrieben.

Oxazolidinonderivate mit anderen Substituenten des Piperidinrings sind z. B. aus der EP 0 635 505 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des vonWillebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Entzündungen, Arteriosklerose verwendet werden. Ferner haben die Verbindungen einen Effekt auf Tumorzellen, indem sie deren Metastasierung hemmen. Somit können sie auch als Anti-Tumor-Mittel eingesetzt werden.

Die Verbindungen eignen sich zudem als antimikrobielle Wirkstoffe, die Infektionen, wie sie beispielsweise

durch Bakterien, Pilze oder Hefen ausgelöst werden, verhindern können. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika.

Die Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man

(a) eine Verbindung der Formel I aus einem ihrer funktionellen Derivate durch Behandeln mit einem solvolysierenden oder hydrogenolysierenden Mittel in Freiheit setzt, oder daß man

(b) eine Verbindung der Formel II

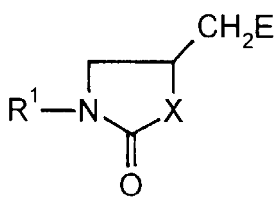

II

worin

E Cl, Br, I oder eine reaktionsfähig veresterte OH-Gruppe bedeutet, und

$R^1$ und X die oben angegebenen Bedeutungen haben mit einer Aminoverbindung der Formel III

$$H\text{-}Y \qquad III$$

worin

Y die oben angegebene Bedeutung hat, umsetzt, oder daß man

(c) eine Verbindung der Formel IV

$$R^1\text{-NH-CH}_2\text{-CH(XH)-CH}_2\text{-Y} \qquad IV$$

worin

$R^1$, X und Y die oben angegebenen Bedeutungen haben, oder eines ihrer reaktionsfähigen Derivate

mit einem reaktiven Derivat der Kohlensäure umsetzt, oder daß man

(d) zur Herstellung einer Guanidinoverbindung der Formel I ($R^1$ = einfach durch $H_2N\text{-C(=NH)-NH}$-substituierter Phenylrest) eine Aminoverbindung entsprechend der Formel I, die jedoch an Stelle des Restes $R^1$ eine Aminophenylgruppe enthält, mit einem amidinierenden Mittel behandelt,

und/oder daß man in einer Verbindung der Formel I einen oder beide Reste $R^1$ und/oder Y in (einen) andere(en) Rest (e) $R^1$ und/oder Y umwandelt und/oder eine Verbindung der Formel I durch Behandeln mit einer Säure oder einer Base in eines ihrer Salze überführt.

Die Verbindungen der Formel I besitzen mindestens ein chirales Zentrum und können daher in mehreren enantiomeren Formen auftreten. Alle diese Formen (z.B. D- und L-Formen) und deren Gemische (z.B. die DL-Formen)

sind in der Formel I eingeschlossen.

Vor- und nachstehend haben die Reste bzw. Parameter X, Y, Z, $R^1$ bis $R^4$, A, k, m, n, p und E die bei den Formeln I oder angegebenen Bedeutungen, falls nicht ausdrücklich etwas anderes angegeben ist. Falls mehrere Gruppen A und/oder Z im Molekül I, II und/oder III vorhanden sind, können sie gleich oder voneinander verschieden sein.

In den vorstehenden Formeln hat die Gruppe A 1-6, vorzugsweise 1,2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl.

X ist O.

Y ist vorzugsweise 3-($R^3$OOC)-azetidino, 3-($R^3OOC-CH_2$-O-)-azetidino, 2-($R^3$OOC-)pyrrolidino, 3-($R^3$OOC-)-pyrrolidino, 2-($R^3$OOC-)-piperidino, 3-($R^3$OOC-)-piperidino, 4-($R^3$OOC-)-piperidino, 2-($R^3OOC-CH_2$-)-piperidino, 3-($R^3OOC-CH_2$-)-piperidino, 4-($R^3OOC-CH_2$-)-piperidino, 4-($R^3OOC-CH_2CH_2$-)-piperidino, 4-Hydroxy-4-($R^3$OOC)-piperidino, 4-Hydroxy-4-($R^3OOC-CH_2$-)-piperidino, 4-Amino-4-($R^3$OOC)-piperidino, 4-Amino-4-($R^3OOC-CH_2$)-piperidino, 3-Oxo-4-($R^3OOC-CH_2$-)-piperidino, 2-($R^3OOC-CH_2$-O-)-piperidino, 3-($R^3OOC-CH_2$-O-)-piperidino, 4-($R^3OOC-CH_2$-O-)piperidino, 1-Oxa-2-oxo-8-azaspiro[4,5]-decan-8-yl, 2-, 3- oder 4-($R^3$OOC)-hexahydroazepino, 4-($R^3OOC-CH_2$-)-piperazino, 4-($R^3OOC-CH_2CH_2$-)piperazino, 2-($R^3$OOC)-piperazino, 3-($R^3$OOC)-piperazino, 4-Benzyl-3-($R^3$OOC)-piperazino.

Z ist vorzugsweise H, ferner bevorzugt A wie Methyl oder Ethyl, Phenyl, Benzyl, Acetyl oder Benzoyl.

$R^1$ ist vorzugsweise ein in 4-Stellung, aber auch in 2- oder 3-Stellung wie angegeben substituierter Phenylrest, im einzelnen bevorzugt 2-, 3- oder (insbesondere) 4-Cyanphenyl, 2-, 3- oder (insbesondere) 4-Aminomethylphenyl, 2-, 3- oder (insbesondere) 4-Dimethylaminomethylphenyl, 2-, 3-oder (insbesondere) 4-Amidinophenyl, 2-, 3- oder 4-Guanidinophenyl, 2-, 3- oder 4-Guanidinomethylphenyl, 2-, 3- oder (insbesondere) 4-Hydroxyamidinophenyl.

$R^2$ ist vorzugsweise $-COOR^3$, $-CH_2COOR^3$ oder $-O-CH_2COOR^3$.

$R^3$ ist vorzugsweise H, Methyl, Ethyl, tert.-Butyl oder Benzyl.

$R^4$ ist vorzugsweise H, Methyl, Ethyl, Benzyl oder $CH_2COOR^3$.

Acyl ist vorzugsweise Alkanoyl mit 1-6 C-Atomen wie Formyl, Acetyl, Propionyl, Butyryl, Isobutyryl, Valeryl oder Capronyl, ferner Benzoyl, Toluyl, 1- oder 2-Naphthoyl oder Phenylacetyl.

Die Parameter k und m sind vorzugsweise 0 oder 1. Der Parameter n ist vorzugsweise 0. Der Parameter p ist vorzugsweise 1.

Unter den Verbindungen der Formel I sind diejenigen bevorzugt, in denen mindestens einer der angegebenen Reste, Gruppen und/oder Parameter eine der angegebenen bevorzugten Bedeutungen hat. Einige Gruppen von bevorzugten Verbindungen sind diejenigen der Formeln Ia bis Id, die der Formel I entsprechen, worin jedoch

in Ia X O bedeutet;

in Ib X O und
$R^1$ Cyanphenyl bedeutet;

in Ic X O und
$R^1$ Aminomethylphenyl bedeutet;

in Id X O und
$R^1$ Amidinophenyl bedeutet.

Weiterhin sind bevorzugt Verbindungen der Formeln Ie sowie Iae, Ibe, Ice und Ide, die den Formeln I, Ia, Ib, Ic und Id entsprechen, worin jedoch zusätzlich

Y 3-$R^2$-azetidino, 2-$R^2$-pyrrolidino, 2-$R^2$-piperidino, 3-$R^2$-piperidino, 4-$R^2$-piperidino, 4-$R^2$-piperazino oder 3-$R^2$-4-$R^4$-piperazino,
$R^2$ $-COOR^3$, $-CH_2COOR^3$ oder $-OCH_2COOR^3$ und
$R^4$ $-CH_2COOR^3$ bedeutet.

Kleinere ausgewählte Gruppen von Verbindungen sind diejenigen der Formeln If und Ig. Sie entsprechen der Formel I, wobei jedoch

in If X O,

Y  3-($R^3$OOC-$CH_2$-O-)-azetidino, 2-($R^3$OOC-)-pyrrolidino, 2-, 3-oder 4-($R^3$OOC-)-piperidino, 4-($R^3$OOC-$CH_2$-)-piperidino, 3-oder 4-($R^3$OOC-$CH_2$-O-)-piperidino, 4-($R^3$OOC-$CH_2$)-piperazino oder 3-($R^3$OOC-)-4-$R^4$-piperazino,
$R^1$  4-Cyanphenyl, 4-Aminomethylphenyl, 4-Amidinophenyl, oder 4-Guanidinomethylphenyl,
$R^3$  H, C1-C4-Alkyl oder Benzyl und
$R^4$  H oder Benzyl bedeuten, und

in Ig X O,

Y  4-($R^3$OOC-)-piperidino oder 4-($R^3$OOC-$CH_2$O-)-piperidino,
$R^1$  4-Cyanphenyl, 4-Aminomethylphenyl oder 4-Amidinophenyl und
$R^3$  H, $C_1$-$C_4$-Alkyl oder Benzyl bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0381033, EP-A1-0462960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere solche, die an Stelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch an Stelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe an Stelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, Sulfoxide wie Dimethylsulfoxid (DMSO), ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1.

Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion einer Verbindung der Formel II mit einer Base der Formel III erhalten werden. Dabei bedient man sich zweckmäßig der an sich bekannten Methoden der N-Alkylierung.

Die Fluchtgruppe E bedeutet vorzugsweise Cl, Br, I, $C_1$-$C_6$-Alkylsulfonyloxy wie Methan- oder Ethansulfonyloxy oder $C_6$-$C_{10}$-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetall-hydroxids oder carbonats wie Natrium-, Kalium- oder Calciumhydroxid, Natrium-, Kalium- oder Calciumcarbonat, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe E von I verschieden ist, empfiehlt sich ein Zusatz eines Iodids wie Kaliumiodid.

Die Ausgangsstoffe der Formel II sind in der Regel neu. Sie können z.B. hergestellt werden durch Reaktion eines substituierten Anilins der Formel $R^1$-$NH_2$ mit einer Verbindung der Formel $R^5CH_2$-$CHR^6$-$CH_2OH$ (worin $R^5$ E, $R^6$ $XR^7$, $R^7$ eine Schutzgruppe, $R^5$ und $R^6$ zusammen auch O bedeuten) zu einer Verbindung der Formel $R^1$-NH-$CH_2$-$CHR^8$-$CH_2OH$ (worin $R^8$ $XR^7$ oder OH bedeutet), gegebenenfalls Abspaltung der Schutzgruppe $R^7$ zu Verbindungen der Formel $R^1$-NH-$CH_2$-CH(XH)-$CH_2OH$, Reaktion mit einem Derivat der Kohlensäure wie Diethylcarbonat zu 3-$R^1$-5-hydroxymethyl-2-oxazolidinonen und Umwandlung der Hydroxymethylgruppe in eine $CH_2E$-Gruppe, z.B. mit $SOCl_2$, $SOBr_2$, Methansulfonylchlorid oder p-Toluolsulfonylchlorid. Die Verbindungen der Formel H-Y (III) sind in der Regel bekannt oder in Analogie zu bekannten Verbindungen herstellbar.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethyl-chlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel $R^1$-NH-$CH_2$-CH(XH)-$CH_2OH$ zu Verbindungen der Formel $R^1$-NH-$CH_2$-CH

(XH)-$CH_2$-E und Reaktion mit Verbindungen der Formel H-Y (III).

Zur Herstellung von Verbindungen der Formel I, worin $R^1$ eine Guanidinophenylgruppe bedeutet, kann man eine entsprechende Aminophenylverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Weiterhin ist es möglich, in einer Verbindung der Formel I einen oder beide der Reste $R^1$ und/oder Y in (einen) andere(n) Rest(e) $R^1$ und/oder Y umzuwandeln.

Insbesondere kann man Cyangruppen zu Aminomethylgruppen reduzieren oder in Amidinogruppen oder Hydroxyamidinogruppen umwandeln, Carboxylgruppen verestern, Estergruppen spalten, Benzylgruppen hydrogenolytisch entfernen, Aminomethylgruppen in Guanidinomethylgruppen überführen.

Eine Reduktion von Cyangruppen zu Aminomethylgruppen gelingt zweckmäßigerweise durch katalytische Hydrierung, z.B. an Raney-Nickel bei Temperaturen zwischen 0 und 100°, vorzugsweise 10 und 30°, und Drucken zwischen 1 und 200 bar, vorzugsweise bei Normaldruck, in einem inerten Lösungsmittel, z.B. einem niederen Alkohol wie Methanol oder Ethanol, zweckmäßig in Gegenwart von Ammoniak. Arbeitet man z.B. bei etwa 20° und 1 bar, so bleiben im Ausgangsmaterial vorhandene Benzylester- oder N-Benzylgruppen erhalten. Will man diese hydrogenolytisch spalten, so verwendet man zweckmäßig einen Edelmetallkatalysator, vorzugsweise Pd-Kohle, wobei man der Lösung eine Säure wie Essigsäure sowie auch Wasser zusetzen kann.

Zur Herstellung eines Amidins der Formel I ($R^1$ = Amidinophenyl) kann man an ein Nitril der Formel I ($R^1$ = Cyanphenyl) Ammoniak anlagern. Die Anlagerung erfolgt bevorzugt mehrstufig, indem man in an sich bekannter Weise a) das Nitril mit $H_2S$ in ein Thioamid umwandelt, das mit einem Alkylierungsmittel, z.B. $CH_3I$, in den entsprechenden S-Alkyl-imidothioester übergeführt wird, welcher seinerseits mit $NH_3$ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithiumbis-(trimethylsilyl)-amid umsetzt und das Produkt anschließend hydrolysiert.

Analog sind die entsprechenden N-Hydroxy-amidine der Formel I ($R^1$ = durch HO-NH-C(=NH)-substituiertes Phenyl) aus den Nitrilen erhältlich, wenn man nach a) oder b), aber mit Hydroxylamin an Stelle von Ammoniak arbeitet.

Zur Veresterung kann man eine Säure der Formel I ($R^3$ = H) mit einem Überschuß eines Alkohols der Formel $R^3$-OH ($R^3$ = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I ($R^3$ = A oder Benzyl), in die entsprechende Säure der Formel I ($R^3$ = H) umgewandelt werden, zweckmäßig durch Solvolyse nach einer der oben angegebenen Methoden, z.B. mit NaOH oder KOH in Wasser-Dioxan bei Temperaturen zwischen 0 und 40°, vorzugsweise 10 und 30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Lauryl-schwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel 1 ($R^3$ = H) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenyl-glycin) gefüllten Säule; als Laufmittel eignet sich z.B. ein Gemisch Hexan/Isopropanol/ Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farbund/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. FAB = ($M^+$ + 1)-Peak im Massenspektrum, erhalten nach der "Fast Atom Bombardment"-Methode.

Beispiel 1

Eine Lösung von 1 g 1-tert.-Butoxycarbonyl-4-(3-(4-cyanphenyl)-2-oxo-5-oxazolidinylmethyl)-piperazin-2-carbonsäureethylester (erhältlich durch Reaktion von 3-(4-Cyanphenyl)-5-brommethyl-2-oxazolidinon mit 1-tert.-Butoxycarbonyl-piperazin-2-carbonsäureethylester nach der in Beispiel 3 beschriebenen Methode) in 12 ml Dichlormethan und 12 ml Trifluoressigsäure wird 1 Std. bei 20° stehengelassen und eingedampft. Man erhält 1-(3-(4-Cyanphenyl)-2-oxo-5-oxazolidinylmethyl)-pipernzin-3-carbonsäureethylester, FAB 359.

Analog erhält man aus 1-tert.-Butoxycarbonyl-4-(3-(4-cyanphenyl)-2-oxo-5-oxazolidinylmethyl)-piperazin-2-carbonsäure bzw. aus deren Benzylester die 1-(3-(4-Cyanphenyl)-2-oxo-5-oxazolidinylmethyl)-piperazin-3-carbonsäure bzw. deren Benzylester.

Beispiel 2

Eine Lösung von 1 g 1-(3-(4-Benzyloxycarbonylaminomethylphenyl)-2-oxo-5-oxazolid inylmethyl)-piperid in-4-oxyessigsäure-tert.-butylester (erhältlich aus 3-(4-Benzyloxycarbonylaminomethylphenyl)-5-chlormethyl-2-oxazolidinon und Piperidin-4-oxyessigsäure-tert.-butylester nach der in Beispiel 3 beschriebenen Methode) in einem Gemisch von 38 ml Methanol, 6 ml Wasser und 6 ml Essigsäure wird an 0,6 g 5%ig. Pd-Kohle bei 20° und 1 bar bis zum Ende der $H_2$-Aufnahme hydriert. Man filtriert, dampft das Filtrat ein und erhält 1-(3-(4-Aminomethylphenyl)-2-oxo-5-oxazolid inylmethyl)-piperidin-4-oxyessigsäu re-tert.-butylester, F. 95-96°, FAB 420.

Beispiel 3

Ein Gemisch von 2,96 g 3-(4-Cyanphenyl)-5-methansulfonyloxymethyl-2-oxazolidinon (F. 162-163°; erhältlich durch Reaktion von 4-Aminobenzonitril mit 2,3-Epoxypropanol zu 4-(2,3-Dihydroxypropyl-amino)-benzonitril (ölig), Umsetzung mit Diethylcarbonat/K-tert.-butylat bei 110° zu 3-(4-Cyanphenyl)-5-hydroxymethyl-2-oxazolidinon (F. 130-131°) und Veresterung mit Methansulfonylchlorid), 1,69 g Piperidin-4-carbonsäureethylester, 70 ml Acetonitril, 1,38 g Kaliumcarbonat und 1,65 g Kaliumiodid wird 25 Std. gekocht. Nach üblicher Aufarbeitung erhält man 1-(3-(4-Cyanphenyl)-2-oxo-5-oxazolidinylmethyl)-piperidin-4-carbonsäureethylester ("IA"), FAB 358.

Analog erhält man:

mit Piperidin-4-carbonsäurebenzylester:
1 -(3-(4-Cyanphenyl)-2-oxo-5-oxazolidinylmethyl)-piperidin-4-carbonsäurebenzylester, F. 96°, FAB 420;
mit Piperidin-4-carbonsäure-tert.-butylester:
1 -(3-(4-Cyanphenyl)-2-oxo-5-oxazolidinylmethyl)-piperidin-4-carbonsäure-tert.-butylester;
mit Piperidin-3-carbonsäureethylester:
1-(3-(4-Cyanphenyl)-2-oxo-5-oxazolidinylmethyl)-piperidin-3-carbonsäureethylester;
mit Piperidin-3-carbonsäurebenzylester:
1-(3-(4-Cyanphenyl)-2-oxo-5-oxazolidinylmethyl)-piperidin-3-carbonsäurebenzylester, FAB 420;
mit Piperidin-3-carbonsäure-tert.-butylester:
1-(3-(4-Cyanphenyl)-2-oxo-5-oxazolidinylmethyl)-piperidin-3-carbonsäure-tert.-butylester;
mit Piperidin-2-carbonsäureethylester:
1-(3-(4-Cyanphenyl)-2-oxo-5-oxazolidinylmethyl)-piperidin-2-carbonsäureethylester;
mit Piperidin-2-carbonsäurebenzylester:
1-(3-(4-Cyanphenyl)-2-oxo-5-oxazolidinylmethyl)-piperidin-2-carbonsäurebenzylester, FAB 420;
mit Piperidin-2-carbonsäure-tert.-butylester:
1-(3-(4-Cyanphenyl)-2-oxo-5-oxazolidinylmethyl)-piperidin-2-carbonsäure-tert.-butylester;
mit Pyrrolidin-2-carbonsäureethylester:
1-(3-(4-Cyanphenyl)-2-oxo-5-oxazolidinylmethyl)-pyrrolidin-2-carbonsäureethylester;
mit Pyrrolidin-2-carbonsäurebenzylester:
1-(3-(4-Cyanphenyl)-2-oxo-5-oxazolidinylmethyl)-pyrrolidin-2-carbonsäurebenzylester;
mit Pyrrolidin-2-carbonsäure-tert.-butylester:
1-(3-(4-Cyanphenyl)-2-oxo-5-oxazolidinylmethyl)-pyrrolidin-2-carbonsäure-tert.-butylester;
mit Piperidin-4-essigsäureethylester:
1-(3-(4-Cyanphenyl)-2-oxo-5-oxazolidinylmethyl)-piperidin-4-essigsäureethylester;
mit Piperidin-4-essigsäurebenzylester:
1-(3-(4-Cyanphenyl)-2-oxo-5-oxazolidinylmethyl)-piperidin-4-essigsäurebenzylester;
mit Piperidin-4-essigsäure-tert.-butylester:
1-(3-(4-Cyanphenyl)-2-oxo-5-oxazolidinylmethyl)-piperidin-4-essigsäure-tert.-butylester;
mit Piperidin-4-oxyessigsäureethylester:
1-(3-(4-Cyanphenyl)-2-oxo-5-oxazolidinylmethyl)-piperidin-4-oxyessigsäureethylester;
mit Piperidin-4-oxyessigsäurebenzylester:
1-(3-(4-Cyanphenyl)-2-oxo-5-oxazolidinylmethyl)-piperidin-4-oxyessigsäurebenzylester;
mit Piperidin-4-oxyessigsäure-tert.-butylester:
1-(3-(4-Cyanphenyl)-2-oxo-5-oxazolidinylmethyl)-piperidin-4-oxyessigsäure-tert.-butylester, F. 88-89°; FAB 416;
mit Piperid in-3-oxyessigsäureethylester:
1-(3-(4-Cyanphenyl)-2-oxo-5-oxazolidinylmethyl)-piperidin-3-oxyessigsäureethylester;
mit Piperidin-3-oxyessigsäurebenzylester:
1-(3-(4-Cyanphenyl)-2-oxo-5-oxazolidinylmethyl)-piperidin-3-oxyessigsäurebenzylester;
mit Piperidin-3-oxyessigsäure-tert.-butylester:
1-(3-(4-Cyanphenyl)-2-oxo-5-oxazolidinylmethyl)-piperidin-3-oxyessigsäure-tert.-butylester;
mit Piperazin-1-essigsäureethylester:
1-(3-(4-Cyanphenyl)-2-oxo-5-oxazolidinylmethyl)-piperazin-4-essigsäureethylester;
mit Piperazin-1-essigsäurebenzylester:
1-(3-(4-Cyanphenyl)-2-oxo-5-oxazolidinylmethyl)-piperazin-4-essigsäurebenzylester;
mit Piperazin-1-essigsäure-tert.-butylester:
1-(3-(4-Cyanphenyl)-2-oxo-5-oxazolidinylmethyl)-piperazin-4-essigsäure-tert.-butylester;
mit Azetidin-3-oxyessigsäureethylester:

1-(3-(4-Cyanphenyl)-2-oxo-5-oxazolidinylmethyl)-azetidin-3-oxyessigsäureethylester;

mit Azetidin-3-oxyessigsäurebenzylester:

1-(3-(4-Cyanphenyl)-2-oxo-5-oxazolidinylmethyl)-azetidin-3-oxyessigsäurebenzylester;

mit Azetidin-3-oxyessigsäure-tert.-butylester:

1-(3-(4-Cyanphenyl)-2-oxo-5-oxazolidinylmethyl)-azetidin-3-oxyessigsäure-tert.-butylester;

mit 1-Benzylpiperazin-2-carbonsäureethylester:

1-Benzyl-4-(3-(4-cyanphenyl)-2-oxo-5-oxazolidinylmethyl)-piperazin 2-carbonsäureethylester;

mit 1-Benzylpiperazin-2-carbonsäurebenzylester:

1-Benzyl-4-(3-(4-cyanphenyl)-2-oxo-5-oxazolidinylmethyl)-piperazin-2-carbonsäurebenzylester;

mit 1-Benzylpiperazin-2-carbonsäure-tert.-butylester:

1-Benzyl-4-(3-(4-cyanphenyl)-2-oxo-5-oxazolidinylmethyl)-piperazin-2-carbonsäure-tert.-butylester.

Analog erhält man aus 3-(4-Cyanphenyl)-5S-methansulfonyloxymethyl-2-oxazolidinon (F. 141-142°; $[\alpha]_D^{20}$ +75,3° (c = 3,9 mg/ml in Methanol); erhältlich durch Reaktion von 4-Aminobenzonitril mit 2R-2,3-Epoxypropanol zu 4-(2S,3-Dihydroxypropylamino)-benzonitril, Umsetzung mit Diethylcarbonat/K-tert.-butylat zu 3-(4-Cyanphenyl)-5S-hydroxymethyl-2-oxazolidinon und Veresterung mit Methansulfonylchlorid) mit Piperidin-4carbonsäurebenzylester:

1-(3-(4-Cyanphenyl)-2-oxo-5S-oxazolidinylmethyl)-piperidin-4-carbonsäurebenzylester, F. 87-88°, $[\alpha]_D^{20}$ 43,0° (c = 9,8 mg/ml in Methanol).

Analog erhält man

mit Piperidin-4-carbonsäureethylester:

1-(3-(4-Cyanphenyl)-2-oxo-5S-oxazolidinylmethyl)-piperidin-4-carbonsäureethylester;

mit Piperidin-4-carbonsäure-tert.-butylester:

1-(3-(4-Cyanphenyl)-2-oxo-5S-oxazolidinylmethyl)-piperidin-4-carbonsäure-tert.-butylester.

Analog erhält man aus 3-(4-Cyanphenyl)-5R-methansulfonyloxymethyl-2-oxazolidinon (erhältlich aus 2S-2,3-Epoxypropanol über 4-(2R,3-Dihydroxypropylamino)-benzonitril und 3-(4-Cyanphenyl)-5R-hydroxymethyl-2-oxazolidinon) den 1-(3-(4Cyanphenyl)-2-oxo-5R-oxazolidinylmethyl)piperidin-4-carbonsäurebenzylester sowie den entsprechenden Ethylester und den entsprechenden tert.-Butylester.

Beispiel 4

Analog Beispiel 3 erhält man aus 3-(4-Dimethylaminomethylphenyl)-5-brommethyl-2-oxazolidinon (erhältlich durch Reaktion von 4-Dimethylaminomethylanilin mit 2,3-Epoxypropanol zu 3-(4-Dimethylaminomethylphenylamino)-1,2-propandiol, Umsetzung mit Diethylcarbonat/K-tert.-butylat zu 3-(4-Dimethylaminomethylphenyl)-5-hydroxymethyl-2-oxazolidinon und Reaktion mit $SOBr_2$) mit Piperidin-4-carbonsäure-tert.-butylester den 1-(3-(4-Dimethylaminomethylphenyl)-2-oxo-5-oxazolidinylmethyl)piperidin-4-carbonsäure-tert.-butylester, FAB 432.

Beispiel 5

Ein Gemisch von 3,31 g 1-(3-(4-Cyanphenyl)-2-hydroxy-propyl)-piperidin-4-carbonsäureethylester (erhältlich durch Reaktion von 3-(4-Cyanphenyl)-1,2-propandiol-1-methansulfonat mit Piperidin-4-carbonsäureethylester), 15 ml Diethylcarbonat und 0,1 g Kalium-tert.-butylat wird 2 Std. bei 110° Badtemperatur gerührt. Man dampft ein, arbeitet wie üblich auf und erhält "IA", FAB 358.

Beispiel 6

Eine Lösung von 201 mg 1-Amidino-3,5-dimethylpyrazol-nitrat in 17 ml Dioxan und 5 ml Wasser wird mit 0,17 ml Ethyl-diisopropylamin versetzt und 15 Min. gerührt. Man gibt dann 375 mg 1-(3-(4-Aminophenyl)-2-oxo-5-oxazolidinylmethyl)-piperid in-4-carbonsäure-tert.-butylester (erhältlich durch Reaktion von 4-Amino-acetanilid mit 2,3-Epoxypropanol zu 4-(2,3-Dihydroxypropylamino)-acetanilid, Umsetzung mit Diethylcarbonat zu 3-(4-Acetamidophenyl)-5-hydroxymethyl-2-oxazolidinon, Umwandlung in das Methansulfonat und Reaktion mit Piperidin-4-carbonsäure-tert.-butylester) hinzu, kocht das Gemisch 45 Std., dampft ein, arbeitet wie üblich auf und erhält 1-(3-(4-Guanidinophenyl)-2-oxo-5-oxazolidinylmethyl)-piperidin-4-carbonsäure-tert.-butylester.

Analog erhält man aus 1-(3-(4-Aminomethylphenyl)-2-oxo-5-oxazolidinylmethyl)-piperidin-4-carbonsäure-tert.-butylester mit 1-Amidino-3,5-dimethylpyrazol-nitrat den 1-(3-(4-Guanidinomethylphenyl)-2-oxo-5-oxazolidinylmethyl)-piperidin-4-carbonsäure-tert.-butylester, FAB 432.

Beispiel 7

Eine Lösung von 1 g "IA" in 40 ml 10%iger methanolischer $NH_3$-Lösung wird an 0,6 g Raney-Ni bei 20° und 1 bar bis zum Ende der $H_2$-Aufnahme hydriert. Nach Filtrieren und Eindampfen erhält man 1-(3-(4-Aminomethylphenyl)-2-oxo-5-oxazolidinylmethyl)-piperidin-4-carbonsäureethylester, FAB 362.

Analog erhält man durch Hydrierung der entsprechenden Nitrile die folgenden 1-(3-(4-Aminomethylphenyl)-2-oxo-5-oxazolidinylmethyl)-piperidine:

- 4-carbonsäure-tert.-butylester
- 3-carbonsäure-ethylester
- 3-carbonsäure-tert.-butylester
- 2-carbonsäure-ethylester
- 2-carbonsäure-tert.-butylester
- 4-essigsäureethylester
- 4-essigsäure-tert.-butylester
- 4-oxyessigsäureethylester
- 4-oxyessigsäure-tert.-butylester, F. 95-96°, FAB 420
- 3-oxyessigsäureethylester
- 3-oxyessigsäure-tert. -butylester;

die folgenden 1-(3-(4-Aminomethylphenyl)-2-oxo-5-oxazolidinylmethyl)pyrrolidine:

- 2-carbonsäureethylester
- 2-carbonsäure-tert.-butylester;

die folgenden 1-(3-(4-Aminomethylphenyl)-2-oxo-5-oxazolidinylmethyl)piperazine:

- 4-essigsäureethylester
- 4-essigsäure-tert.-butylester;

die folgenden 1-(3-(4-Aminomethylphenyl)-2-oxo-5-oxazolidinylmethyl)azetidine:

- 3-oxyessigsäureethylester
- 3-oxyessigsäure-tert.-butylester;

die folgenden 1-(3-(4-Aminomethylphenyl)-2-oxo-5-oxazolidinylmethyl)-4-benzylpiperazine:

- 3-carbonsäureethylester
- 3-carbonsäure-tert.-butylester;

die folgenden 1-(3-(4-Aminomethylphenyl)-2-oxo-5S-oxazolidinylmethyl)-piperidine:

- 4-carbonsäureethylester
- 4-carbonsäure-tert.-butylester;

die folgenden 1-(3-(4-Aminomethylphenyl)-2-oxo-5R-oxazolidinylmethyl)piperidine:

- 4-carbonsäureethylester
- 4-carbonsäure-tert.-butylester.

Beispiel 8

In eine Lösung von 3,57 g "IA" in 50 ml Pyridin und 6,6 ml Triethylamin wird bei -10° $H_2S$ eingeleitet (45 Min.). Anschließend rührt man 14 Std. bei 20°, dampft ein, löst den Rückstand in 50 ml Aceton und versetzt mit 9 ml Methyliodid. Nach 6stündigem Rühren bei 20° filtriert man ab, wäscht den Rückstand mit wenig Aceton, löst in 30 ml Methanol, gibt 4,6 g Ammoniumacetat hinzu und rührt 30 Std. bei 20°. Der erhaltene 1-(3-(4-Amidinophenyl)-2-oxo-5-oxazolidinylmethyl)-piperidin-4-carbonsäureethylester wird abfiltriert und an Kieselgel chromatographiert (Dichlormethan/Methanol/Essigsäure 70:30:2); F. 200° (Zers.); FAB 375.

Analog erhält man aus den ensprechenden Nitrilen die folgenden 1-(3-(4-Amidinophenyl)-2-oxo-5-oxazolidinylmethyl)-piperidine:

- 4-carbonsäurebenzylester, F. 204° (Zers.); FAB 437; Dihydrochlorid, F. 182°
- 4-carbonsäure-tert.-butylester
- 3-carbonsäureethylester
- 3-carbonsäurebenzylester, Acetat, FAB 437
- 3-carbonsäure-tert.-butylester
- 2-carbonsäureethylester
- 2-carbonsäurebenzylester, FAB 437
- 2-carbonsäure-tert.-butylester
- 4-essigsäureethylester
- 4-essigsäurebenzylester, Acetat, F. 206°
- 4-essigsäure-tert.-butylester
- 4-oxyessigsäureethylester
- 4-oxyessigsäurebenzylester
- 4-oxyessigsäure-tert.-butylester, F. 187° (Zers.); FAB 433
- 3-oxyessigsäureethylester
- 3-oxyessigsäurebenzyleser
- 3-oxyessigsäure-tert.-butylester;

die folgenden 1-(3-(4-Amidinophenyl)-2-oxo-5-oxazolidinylmethyl)pyrrolidine:

- 2-carbonsäureethylester
- 2-carbonsäurebenzylester
- 2-carbonsäure-tert.-butylester;

die folgenden 1-(3-(4-Amidinophenyl)-2-oxo-5-oxazolidinylmethyl)piperazine:

- 4-essigsäureethylester
- 4-essigsäurebenzylester, FAB 452
- 4-essigsäure-tert.-butylester;

die folgenden 1-(3-(4-Amidinophenyl)-2-oxo-5-oxazolidinylmethyl)azetidine:

- 3-oxyessigsäureethylester
- 3-oxyessigsäurebenzylester
- 3-oxyessigsäure-tert.-butylester;

die folgenden 1-(3-(4-Amidinophenyl)-2-oxo-5-oxazolidinylmethyl)-4-benzylpiperazine:

- 3-carbonsäureethylester
- 3-carbonsäurebenzylester
- 3-carbonsäure-tert.-butylester;

die folgenden 1-(3-(4-Amidinophenyl)-2-oxo-5S-oxazolidinylmethyl)piperidine:

- 4-carbonsäureethylester
- 4-carbonsäurebenzylester
- 4-carbonsäure-tert.-butylester;

die folgenden 1-(3-(4-Amidinophenyl)-2-oxo-5R-oxazolidinylmethyl)piperidine:

- 4-carbonsäureethylester
- 4-carbonsäurebenzylester
- 4-carbonsäure-tert.-butylester.

Beispiel 9

Analog Beispiel 8, aber mit einer äquivalenten Menge Hydroxylammoniumacetat an Stelle von Ammoniumacetat, erhält man aus "IA" den 1-(3-(4-Hydroxyamidinophenyl)-2-oxo-5-oxazolidinylmethyl)-piperidin-4-carbonsäureethylester.

Beispiel 10

Man löst 1g 1-(3-(4-Cyanphenyl)-2-oxo-5-oxazolidinylmethyl)-piperidin-4-carbonsäure-tert.-butylester in 12 ml Dichlormethan, gibt 12 ml Trifluoressigsäure hinzu, läßt 5 Min. stehen, dampft ein, arbeitet wie üblich auf und erhält 1-(3-(4-Cyanphenyl)-2-oxo-5-oxazolidinylmethyl)-piperidin-4-carbonsäure, FAB 330.

Analog erhält man aus den entsprechenden tert.-Butylestern die folgenden Säuren:

1-(3-(4-Cyanphenyl)-2-oxo-5-oxazolidinylmethyl)-piperid in-3-carbonsäure
1-(3-(4-Cyanphenyl)-2-oxo-5-oxazolidinylmethyl)-piperidin-2-carbonsäure
1-(3-(4-Cyanphenyl)-2-oxo-5-oxazolidinylmethyl)-pyrrolidin-2-carbonsäure
1-(3-(4-Cyanphenyl)-2-oxo-5-oxazolidinylmethyl)-piperidin-4-essigsäure
1-(3-(4-Cyanphenyl)-2-oxo-5-oxazolidinylmethyl)-piperidin-4-oxyessigsäure
1-(3-(4-Cyanphenyl)-2-oxo-5-oxazolidinylmethyl)-piperidin-3-oxyessigsäure
1-(3-(4-Cyanphenyl)-2-oxo-5-oxazolidinylmethyl)-piperazin-4-essigsäure
1-(3-(4-Cyanphenyl)-2-oxo-5-oxazolidinylmethyl)-azetidin-3-oxyessigsäure 1-Benzyl-4-(3-(4-cyanphenyl)-2-oxo-5-oxazolidinylmethyl)-piperazin-2-car-bonsäure
1-(3-(4-Aminomethylphenyl)-2-oxo-5-oxazolidinylmethyl)-piperidin-4-carbonsäure, F. 190° (Zers.); FAB 334
1-(3-(4-Aminomethylphenyl)-2-oxo-5-oxazolidinylmethyl)-piperidin-3-carbonsäure
1-(3-(4-Aminomethylphenyl)-2-oxo-5-oxazolidinylmethyl)-piperidin-2-carbonsäure
1-(3-(4-Aminomethylphenyl)-2-oxo-5-oxazolidinylmethyl)-pyrrolidin-2-carbonsäure
1-(3-(4-Aminomethylphenyl)-2-oxo-5-oxazolidinylmethyl)-piperidin-4-essigsäure
1-(3-(4-Aminomethylphenyl)-2-oxo-5-oxazolidinylmethyl)-piperidin-4-oxyessigsäure, F. 135°; FAB 364
1-(3-(4-Aminomethylphenyl)-2-oxo-5-oxazolidinylmethyl)-piperidin-3-oxyessigsäure
1-(3-(4-Aminomethylphenyl)-2-oxo-5-oxazolidinylmethyl)-piperazin-4-essigsäure
1-(3-(4-Aminomethylphenyl)-2-oxo-5-oxazolidinylmethyl)-azetidin-3-oxyessigsäure
1-Benzyl-4-(3-(4-aminomethylphenyl)-2-oxo-5-oxazolidinylmethyl)-piperazin-2-carbonsäure
1-(3-(4-Amidinophenyl)-2-oxo-5-oxazolidinylmethyl)-piperidin-4-carbonsäure, F. 265° (Zers.); FAB 347; Dihydrochlorid, F. 142°
1-(3-(4-Amidinophenyl)-2-oxo-5-oxazolidinylmethyl)-piperidin-3-carbonsäure
1-(3-(4-Amidinophenyl)-2-oxo-5-oxazolidinylmethyl)-piperidin-2-carbonsäure, F. 198°; FAB 347
1-(3-(4-Amidinophenyl)-2-oxo-5-oxazolidinylmethyl)-pyrrolidin-2-carbonsäure
1-(3-(4-Amidinophenyl)-2-oxo-5-oxazolidinylmethyl)-piperidin-4-essigsäure, F. 256°
1-(3-(4-Amidinophenyl)-2-oxo-5-oxazolidinylmethyl)-piperidin-4-oxyessigsäure, Öl, FAB 377
1-(3-(4-Amidinophenyl)-2-oxo-5-oxazolidinylmethyl)-piperidin-3-oxyessigsäure, F. 167-168°
1-(3-(4-Amidinophenyl)-2-oxo-5-oxazolidinylmethyl)-piperazin-4-essigsäure, FAB 362
1-(3-(4-Amidinophenyl)-2-oxo-5-oxazolidinylmethyl)-azetidin-3-oxyessigsäure
1-Benzyl-4-(3-(4-amidinophenyl)-2-oxo-5-oxazolidinylmethyl)-piperazin-2-carbonsäure
1-(3-(4-Guanidinophenyl)-2-oxo-5-oxazolidinylmethyl)-piperidin-4-carbonsäure
1-(3-(4-Guanidinomethylphenyl)-2-oxo-5-oxazolidinylmethyl)-piperidin-4-carbonsäure, FAB 376
1-(3-(4-Cyanphenyl)-2-oxo-5S-oxazolidinylmethyl)-piperidin-4-carbonsäure
1-(3-(4-Cyanphenyl)-2-oxo-5R-oxazolidinylmethyl)-piperidin-4-carbonsäure
1-(3-(4-Aminomethylphenyl)-2-oxo-5S-oxazolidinylmethyl)-piperidin-4-carbonsäure
1-(3-(4-Aminomethylphenyl)-2-oxo-5R-oxazolidinylmethyl)-piperidin-4-carbonsäure
1-(3-(4-Amidinomethylphenyl)-2-oxo-5S-oxazolidinylmethyl)-piperidin-4-carbonsäure
1-(3-(4-Amidinomethylphenyl)-2-oxo-5R-oxazolidinylmethyl)-piperidin-4-carbonsäure
1-(3-(4-Dimethylaminomethylphenyl)-2-oxo-5-oxazolidinylmethyl)-piperidin-4-carbonsäure.

Beispiel 11

Eine Lösung von 1 g 1-(3-(4-Amidinophenyl)-2-oxo-5-oxazolidinylmethyl)piperidin-4-carbonsäurebenzylester in 100 ml Methanol, 17 ml Essigsäure und 17 ml Wasser wird an 0,7 g 5%ig. Pd-Kohle bei 20° und 1 bar bis zum Stillstand der $H_2$-Aufnahme hydriert. Man filtriert, dampft ein, verreibt mit Diethylether und erhält 1-(3-(4-Amidinophenyl)-2-oxo-5-oxazolidinylmethyl)-piperidin-4-carbonsäure, F. 265°; FAB 347; Dihydrochlorid, F. 142°.

Beispiel 12

Analog Beispiel 11 erhält man durch Hydrierung von 1-Benzyl-4-(3-(4-cyanphenyl)-2-oxo-5-oxazolidinylmethyl)-piperazin-2-carbonsäure ("IB"; oder von "IB"-benzylester) die 1-(3-(4-Aminomethylphenyl)-2-oxo-5-oxazolidinylmethyl)-piperazin-3-carbonsäure.

Die gleiche Substanz ist analog durch Hydrierung von 1-Benzyl-4-(3-(4-aminomethylphenyl)-2-oxo-5-oxazolidinylmethyl)-piperazin-2-carbonsäure (oder von deren Benzylester) erhältlich.

Analog erhält man aus "IB"-ethylester bzw. "IB"-tert.-butylester den 1-(3-(4-Aminomethylphenyl)-2-oxo-5-oxazolidinylmethyl)-piperazin-3-carbonsäureethylester bzw. den 1-(3-(4-Aminomethylphenyl)-2-oxo-5-oxazolidinylmethyl)piperazin-3-carbonsäure-tert.-butylester.

Beispiel 13

Analog Beispiel 3 erhält man aus 3-(4-Cyanphenyl)-5-methansulfonyloxymethyl-2-oxazolidinon:

mit Piperidin-3-essigsäurebenzylester:
 1-(3-(4-Cyanphenyl)-2-oxo-5-oxazolidinylmethyl)-piperidin-3-essigsäurebenzylester, FAB 434
mit 3-(1-Piperazinyl)-propionsäuremethylester:
 3-(4-(3-(4-Cyanphenyl)-2-oxo-5-oxazolidinylmethyl)-1-piperazinyl)propionsäuremethylester, FAB 373
mit 3-(1-Piperazinyl)-propionsäureethylester:
 3-(4-(3-(4-Cyanphenyl)-2-oxo-5-oxazolidinylmethyl)-1-piperazinyl)propionsäureethylester, FAB 387
mit 2-Oxopiperazin-1-essigsäureethylester:
 1-(3-(4-Cyanphenyl)-2-oxo-5-oxazolidinylmethyl)-3-oxo-piperazin-4-essigsäureethylester, FAB 387
mit 3-(2-Oxo-1-piperazinyl)-propionsäureethylester:
 3-(4-(3-(4-Cyanphenyl)-2-oxo-5-oxazolidinylmethyl)-2-oxo-1-piperazinyl)-propionsäureethylester, FAB 401
mit 4-Hydroxy-piperidin-4-carbonsäureethylester:
 1-(3-(4-Cyanphenyl)-2-oxo-5-oxazolidinylmethyl)-4-hydroxy-piperidin-4-carbonsäureethylester, FAB 374
mit 4-Hydroxy-piperidin-4-esigsäureethylester:
 1-(3-(4-Cyanphenyl)-2-oxo-5-oxazolidinylmethyl)-4-hydroxy-piperidin-4-essigsäureethylester, FAB 388
mit 1-Oxa-8-azaspiro[4,5]decan-2-on (= Lacton der 3-(4-Hydroxy-4-piperidinyl)-propionsäure):
 8-(3-(4-Cyanphenyl)-2-oxo-5-oxazolidinylmethyl)-1-oxa-8-azaspiro[4,5]decan-2-on, FAB 356.

Analog erhält man aus 3-(4-Cyanphenyl)-5R- bzw. -5S-methansulfonyloxymethyl-2-oxazolidinon:

mit Piperidin-4-carbonsäurebenzylester:
 1-(3-(4-Cyanphenyl)-2-oxo-5R-oxazlidinylmethyl)-piperidin-4-carbonsäurebenzylester
 1-(3-(4-Cyanphenyl)-2-oxo-5S-oxazolid inylmethyl)-piperidin-4-carbonsäurebenzylester
mit Piperazin-4-essigsäureethylester:
 1-(3-(4-Cyanphenyl)-2-oxo-5R-oxazolidinylmethyl)-piperazin-4-essigsäureethylester, FAB 373
 1-(3-(4-Cyanphenyl)-2-oxo-5S-oxazolidinylmethyl)-piperazin-4-essigsäureethylester, FAB 373
mit 3-(1-Piperazinyl)-propionsäurebenzylester:
 3-(4-(3-(4-Cyanphenyl)-2-oxo-5R-oxazolidinylmethyl)-1-piperazinyl)propionsäurebenzylester, F. 90°
 3-(4-(3-(4-Cyanphenyl)-2-oxo-5S-oxazolidinylmethyl)-1-piperazinyl)propionsäurebenzylester, F. 90°.

Beispiel 14

Analog Beispiel 8 erhält man aus den entsprechenden Nitrilen (siehe Beispiel 13):

1-(3-(4-Amidinophenyl)-2-oxo-5-oxazolidinylmethyl)-piperidin-3-essigsäurebenzylester, Diacetat, F. 287-288°
3-(4-(3-(4-Amidinophenyl)-2-oxo-5-oxazolidinylmethyl)-1-piperazinyl)propionsäureethylester, F. 206°
1-(3-(4-Amidinophenyl)-2-oxo-5-oxazolidinylmethyl)-3-oxo-piperazin-4-essigsäureethylester, F. 224°
3-(4-(3-(4-Amidinophenyl)-2-oxo-5-oxazolidinylmethyl)-2-oxo-1-piperazinyl)-propionsäureethylester, FAB 418
1-(3-(4-Amidinophenyl)-2-oxo-5-oxazolidinylmethyl)-4-hydroxy-piperidin-4-carbonsäureethyelster
1-(3-(4-Amidinophenyl)-2-oxo-5-oxazolidinylmethyl)-4-hydroxypiperidin-4-essigsäureethylester, Acetat, F. 108°
8-(3-(4-Amidinophenyl)-2-oxo-5-oxazolidinylmethyl)-1-oxa-8-azaspiro[4,5]decan-2-on, FAB 373
1-(3-(4-Amidinophenyl)-2-oxo-5R-oxazolidinylmethyl)-piperidin-4-carbonsäurebenzylester, Acetat, F. 225° $[\alpha]_D^{20}$

+40.5° (c = 1, Methanol)
1-(3-(4-Amidinophenyl)-2-oxo-5S-oxazolidinylmethyl)-piperid in-4-carbonsäurebenzylester, Acetat, F. 225°; $[\alpha]_D^{20}$ -41° (c = 1, Methanol)
1-(3-(4-Amidinophenyl)-2-oxo-5R-oxazolidinylmethyl)-piperazin-4-essigsäureethylester, FAB 390
1-(3-(4-Amidinophenyl)-2-oxo-5S-oxazolidinylmethyl)-piperazin-4-essigsäureethylester, FAB 390.

Beispiel 15

Analog Beispiel 9 erhält man aus den entsprechenden Nitrilen:

1-(3-(4-Hydroxyamidinophenyl)-2-oxo-5-oxazolidinylmethyl)-4-hydroxy-piperidin-4-essigsäureethylester, Acetat, F. 178-180°
3-(4-(3-(4-Hydroxyamidinophenyl)-2-oxo-5-oxazolidinylmethyl)-1-piperazinyl)-propionsäuremethylester, F. 202°
3-(4-(3-(4-Hydroxyamidinophenyl)-2-oxo-5R-oxazolidinylmethyl)-1-piperazinyl)-propionsäurebenzylester, F. 159°
3-(4-(3-(4-Hydroxyamidinophenyl)-2-oxo-5S-oxazolidinylmethyl)-1-piperazinyl)-propionsäurebenzylester, F. 159°.

Beispiel 16

Ein Gemisch von 1 g 3-(4-(3-(4-Amidinophenyl)-2-oxo-5-oxazolidinylmethyl)-1-piperazinyl)-propionsäureethylester, 0,2 g NaOH, 8 ml Dioxan und 2 ml Wasser wird bei 20° 5 Std. gerührt. Nach Ansäuern und üblicher Aufarbeitung erhält man 3-(4-(3-(4-Amidinophenyl)-2-oxo-5-oxazolidinylmethyl)-1-piperazinyl)-propionsäure, F. 269°.
Analog erhält man durch Verseifung der entsprechenden Ester:

1-(3-(4-Amidinophenyl)-2-oxo-5-oxazolidinylmethyl)-3-oxo-piperazin-4-essigsäure, Monohydrat, F. 261°
3-(4-(3-(4-Amidinophenyl)-2-oxo-5-oxazolidinylmethyl)-2-oxo-1-piperazinyl)-propionsäure, FAB 390
1-(3-(4-Amidinophenyl)-2-oxo-5-oxazolidinylmethyl)-4-hydroxy-piperidin-4-carbonsäure
1-(3-(4-Amidinophenyl)-2-oxo-5-oxazolidinylmethyl)-4-hydroxy-piperidin-4-essigsäure, F. 230°
1-(3-(4-Amidinophenyl)-2-oxo-5R-oxazolidinylmethyl)-piperazin-4-essigsäure, Acetat, FAB 362; $[\alpha]_D^{20}$ -28,0° (c = 1, DMSO)
1-(3-(4-Amidinophenyl)-2-oxo-5S-oxazolidinylmethyl)-piperazin-4-essigsäure, Acetat, FAB 362; $[\alpha]_D^{20}$ +24,0° (c = 1, DMSO)

Beispiel 17

a) Man löst 1 g 1-(3-(4-Cyanphenyl)-2-oxo-5-oxazolidinylmethyl)-4-methansulfonyloxy-piperidin-4-essigsäureethylester (erhältlich aus der 4-Hydroxy-verbindung (siehe Beispiel 13) mit Methansulfonylchlorid/Pyridin) in einer 2%igen $NH_3$-Lösung in 10 ml eines 1:1-Gemisches von Ethanol und THF und läßt 2 Std. bei 20° stehen. Man dampft ein, arbeitet wie üblich auf und erhält 1-(3-(4-Cyanphenyl)-2-oxo-5-oxazolidinylmethyl)-4-amino-piperidin-4-essigsäureethylester, FAB 387.

b) Analog Beispiel 8 erhält man daraus den 1-(3-(4-Amidinophenyl)-2-oxo-5-oxazolidinylmethyl)-4-amino-piperidin-4-essigsäureethylester, FAB 404.

c) Analog Beispiel 16 erhält man daraus durch Verseifung 1-(3-(4-Amidinophenyl)-2-oxo-5-oxazolidinylmethyl)-4-amino-piperidin-4-essigsäure, FAB 376.

Beispiel 18

a) Analog Beispiel 17a) erhält man aus 1-(3-(4-Cyanphenyl)-2-oxo-5-oxazolid inylmethyl)-4-methansulfonyloxy-piperidin-4-carbonsäureethylester (erhältlich aus der 4-Hydroxy-verbindung (siehe Beispiel 13) mit Methansulfonylchlorid/Pyridin) mit $NH_3$ den 1-(3-(4-Cyanphenyl)-2-oxo-5-oxazolidinylmethyl)-4-amino-piperidin-4-carbonsäureethylester, FAB 373.

b) Analog Beispiel 8 erhält man daraus den 1-(3-(4-Amidinophenyl)-2-oxo-5-oxazolidinylmethyl)-4-amino-piperidin-4-carbonsäureethylester, FAB 390.

c) Analog Beispiel 16 erhält man daraus durch Verseifung 1-(3-(4-Amidinophenyl)-2-oxo-5-oxazolidinylmethyl)-4-amino-piperidin-4-carbonsäure, FAB 362

Beispiel 19

Eine Lösung von 1 g 3-(4-(3-(4-Hydroxyamidinophenyl)-2-oxo-5R- bzw.- 5S-oxazolidinylmethyl)-1-piperazinyl)-propionsäurebenzylester (siehe Beispiel 15) in 30 ml Essigsäure unter Zusatz von 1 ml Acetanhydrid wird an 0,2 g 10%ig. Pd-C bei 20° und 1 bar bis zur Aufnahme der berechneten Menge Wasserstoff hydriert. Man filtriert, arbeitet wie üblich auf und erhält:

3-(4-(3-(4-Amidinophenyl)-2-oxo-5R-oxazolidinylmethyl)-1-piperazinyl)-propionsäure, Acetat, F. 200-220° (Zers.); $[\alpha]_D^{20}$ +9° (c = 0,5, DMSO)

bzw.

3-(4-(3-(4-Amidinophenyl)-2-oxo-5S-oxazolidinylmethyl)-1-piperazinyl)-propionsäure, Acetat, F. 200-220° (Zers.); $[\alpha]_D^{20}$ -8° (c = 0,5, DMSO).

Die nachfolgenden Beispiele betreffen pharmazeutische Zubereitungen.

**Beispiel A: Tabletten**

Ein Gemisch von 1 kg Wirkstoff der Formel I, 4 kg Lactose, 1,2 kg Maisstärke, 200 g Talk und 100 g Magnesiumstearat wird in üblicher Weise zu Tabletten verpreßt, derart, daß jede Tablette 100 mg Wirkstoff enthält.

**Beispiel B: Dragees**

Analog Beispiel A werden Tabletten gepreßt, die anschließend in üblicher Weise mit einem Überzug aus Saccharose, Maisstärke, Talk, Tragant und Farbstoff überzogen werden.

**Beispiel C: Kapseln**

500 g Wirkstoff der Formel I werden in üblicher Weise in Hartgelatinekapseln gefüllt, so daß jede Kapsel 500 mg Wirkstoff enthält.

**Beispiel D: Injektionsgläser**

Eine Lösung von 100 g Wirkstoff der Formel I in 4 l zweifach destilliertem Wasser wird mit 2 n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

**Beispiel E: Suppositorien**

Man schmilzt ein Gemisch von 50 g Wirkstoff der Formel I mit 10 g Sojalecithin und 140 g Kakaobutter, gießt in Formen und läßt erkalten. Jedes Suppositorium enthält 250 mg Wirkstoff.

## Patentansprüche

1. Verbindungen der Formel I

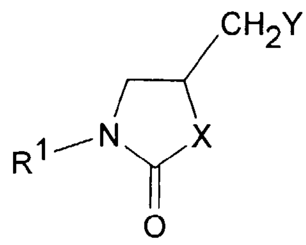

I

worin

X O,

Y einen unsubstituierten oder einen einfach durch $R^2$ substituierten Aziridino-, Azetidino-, Pyrrolidino-, Piperidino-, 1-Oxa-8-azaspiro[4,5]decan-8-yl-, Hexahydroazepino- oder 4-$R^4$-piperazino-rest, rest, der zusätzlich durch eine Gruppe OZ, SZ, oder $N(Z)_2$ und/oder durch Carbonylsauerstoff substituiert sein kann,

Z jeweils H, A, Phenyl-$C_kH_{2k}$- oder Acyl mit 1-11 C-Atomen,

$R^1$ einen einfach durch CN, $H_2N$-$CH_2$-, $(A)_2N$-$CH_2$-, $H_2N$-C(=NH)-, $H_2N$-C(=NH)-NH-, $H_2N$-C(=NH)-NH-$CH_2$-, HO-NH-C(=NH)- oder HO-NH-C(=NH)-NH substituierten Phenylrest,

$R^2$ -$C_mH_{2m}$-$COOR^3$ oder -$C_nH_{2n}$-O-$C_pH_{2p}$-$COOR^3$,

$R^3$ H, A oder Benzyl,

$R^4$ H, A, Benzyl oder -$C_mH_{2m}$-$COOR^3$,

A jeweils Alkyl mit 1-6 C-Atomen,

k und m jeweils 0, 1, 2 oder 3,

n 0, 1 oder 2, und

p 1, 2 oder 3 bedeuten,

sowie deren Salze.

**2.** 1-(3-(4-Amidinophenyl)-2-oxo-5-oxazolidinylmethyl)-piperidin-4-carbonsäure, nach Anspruch 1.

**3.** Verfahren zur Herstellung einer Verbindung der Formel I nach Patentanspruch 1 sowie von ihren Salzen, dadurch gekennzeichnet, daß man

(a) eine Verbindung der Formel I aus einem ihrer funktionellen Derivate durch Behandeln mit einem solvolysierenden oder hydrogenolysierenden Mittel in Freiheit setzt, oder daß man

(b) eine Verbindung der Formel II

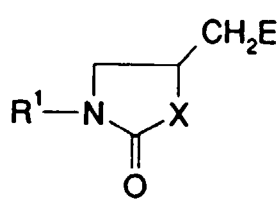

II

worin

E Cl, Br, I oder eine reaktionsfähig veresterte OH-Gruppe bedeutet, und

$R^1$ und X die in Anspruch 1 angegebenen Bedeutungen haben

mit einer Aminoverbindung der Formel III

$$H\text{-}Y \qquad III$$

worin

Y die in Anspruch 1 angegebene Bedeutung hat,

umsetzt, oder daß man

(c) eine Verbindung der Formel IV

$$R^1\text{-}NH\text{-}CH_2\text{-}CH(XH)\text{-}CH_2\text{-}Y \qquad IV$$

worin

$R^1$, X und Y die in Anspruch 1 angegebenen Bedeutungen haben, oder eines ihrer reaktionsfähigen Derivate

mit einem reaktiven Derivat der Kohlensäure umsetzt, oder daß man

(d) zur Herstellung einer Guanidinoverbindung der Formel I ($R^1$ = einfach durch $H_2N$-C(=NH)-NH-substtuierter Phenylrest) eine Aminoverbindung entsprechend der Formel I, die jedoch an Stelle des Restes $R^1$ eine Aminophenylgruppe enthält, mit einem amidinierenden Mittel behandelt,

und/oder daß man in einer Verbindung der Formel I einen oder beide Reste $R^1$ und/oder Y in (einen) andere(en) Rest(e) $R^1$ und/oder Y umwandelt und/oder eine Verbindung der Formel I durch Behandeln mit einer Säure oder einer Base in eines ihrer Salze überführt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Salze.

**6.** Verwendung von Verbindungen der Formel I nach Anspruch 1 oder von deren physiologisch unbedenklichen Salzen zur Herstellung eines Arzneimittels.

**7.** Verwendung von Verbindungen der Formel I nach Anspruch 1 oder von deren physiologisch unbedenklichen Salzen bei der Bekämpfung von Thrombosen, Herzinfarkten, Apoplexie, Osteoporose, Arteriosklerose, Entzündungen und/oderTumoren.

## Claims

**1.** Compounds of the formula I

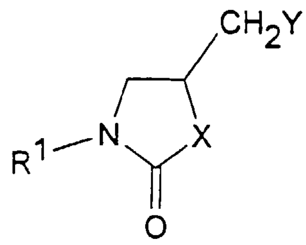

I

in which

| | |
|---|---|
| X | is O, |
| Y | is an aziridino, azetidino, pyrrolidino, piperidino, 1-oxa-8-azaspiro[4.5]dec-9-yl, hexahydroazepino or 4-$R^4$-piperazino radical which is unsubstituted or substituted once by $R^2$, which can additionally be substituted by an OZ, SZ or $N(Z)_2$ group and/or by carbonyl oxygen, |
| Z | is in each case H, A, phenyl-$C_kH_{2k}$ or acyl having 1-11 C atoms, |
| $R^1$ | is a phenyl radical which is substituted once by CN, $H_2N-CH_2-$, $(A)_2N-CH_2-$, $H_2N-C(=NH)-$, $H_2N-C(=NH)-NH-$, $H_2N-C(=NH)-NH-CH_2-$, HO-NH-C(=NH)- or HO-NH-C(=NH)-NH, |
| $R^2$ | is $-C_mH_{2m}-COOR^3$ or $-C_nH_{2n}-O-C_pH_{2p}-COOR^3$, |
| $R^3$ | is H, A or benzyl, |
| $R^4$ | is H, A, benzyl or $-C_mH_{2m}-COOR^3$, |
| A | is in each case alkyl having 1-6 C atoms, |
| k | and m are in each case 0, 1, 2 or 3, |
| n | is 0, 1 or 2, and |
| p | is 1, 2 or 3, |

and salts thereof.

**2.** 1-(3-(4-Amidinophenyl)-2-oxo-5-oxazolidinylmethyl)piperidine-4-carboxylic acid according to Claim 1.

**3.** Process for preparing a compound of the formula I according to Patent Claim 1, and its salts, characterized in that

(a) a compound of the formula I is liberated from one of its functional derivatives by treating with a solvolysing or hydrogenolysing agent, or in that
(b) a compound of the formula II

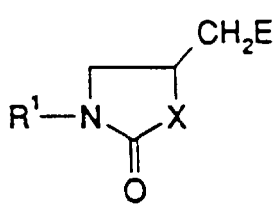

II

in which

E is Cl, Br, I, or a reactive esterified OH group, and
$R^1$ and X have the meanings given in Claim 1,

is reacted with an amino compound of the formula III

$$H\text{-}Y \qquad III$$

in which

Y has the meaning given in Claim 1,

or in that
(c) a compound of the formula IV

$$R^1\text{-NH-CH}_2\text{-CH(XH)-CH}_2\text{-Y} \qquad \text{IV}$$

in which

$R^1$, X and Y have the meanings given in Claim 1, or one of its reactive derivatives,

is reacted with a reactive derivative of carbonic acid, or in that
(d) in order to prepare a guanidino compound of the formula I ($R^1$ = phenyl radical substituted once by $H_2N$-C(=NH)-NH), an amino compound corresponding to the formula I, which, however, contains an aminophenyl group in place of the radical $R^1$, is treated with an amidinating agent,

and/or in that, in a compound of the formula I, one or both radicals $R^1$ and/or Y is/are transformed into (an)other radical(s) $R^1$ and/or Y, and/or a compound of the formula I is converted into one of its salts by treatment with an acid or a base.

**4.** Process for preparing pharmaceutical formulations, characterized in that a compound of the formula I according to Claim 1 and/or one of its physiologically harmless salts is/are brought into a suitable dosage form together with at least one solid, liquid or semi-liquid excipient or auxiliary agent.

**5.** Pharmaceutical formulation, characterized by a content of at least one compound of the formula I according to Claim 1 and/or one of its physiologically harmless salts.

**6.** Use of compounds of the formula I according to Claim 1, or of their physiologically harmless salts, for preparing a medicament.

**7.** Use of compounds of the formula I according to Claim 1, or of their physiologically harmless salts, in the control of thromboses, cardiac infarctions, stroke, osteoporosis, arteriosclerosis, inflammations and/or tumours.

## Revendications

**1.** Composés de formule I

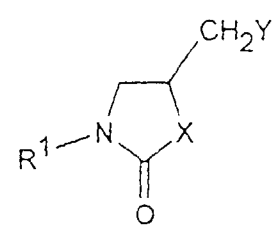

où

| | |
|---|---|
| X | représente O, |
| Y | un reste aziridino, azétidino, pyrrolidino, pipéridino, 1-oxa-8-azaspiro[4,5] décan-8-yle, hexahydroazépino ou 4-$R^4$-pipérazino, non substitués ou monosubstitués par $R^2$ et pouvant être substitués en plus par un groupe OZ, SZ ou $N(Z)_2$ et/ou par l'oxygène du carbonyle, |
| Z | H, A, un phényle-$C_kH_{2k}$- ou un acyle comportant 1 a 11 atomes de C, |
| $R^1$ | un reste phényle monosubstitué par CN, $H_2N$-$CH_2$, $(A)_2N$-$CH_2$-, $H_2N$-C(=NH)-, $H_2N$-C(=NH)-NH-, $H_2N$- |

C(=NH)-NH-$CH_2$-, HO-NH-C(=NH)ou HO-NH-C(=NH)-NH,
$R^2$ $-C_mH_{2m}-COOR^3$ ou $-C_nH_{2n}-O-C_pH_{2p}-COOR^3$,
$R^3$ H, A ou le benzyle,
$R^4$ H, A, le benzyle ou $-C_mH_{2m}-COOR^3$,
A un alkyle comportant 1 à 6 atomes de C,
k et m sont égaux à 0, 1, 2 ou 3,
n est égal à 0, 1 ou 2 et
p à 1, 2 ou 3,

ainsi que leurs sels.

**2.** L'acide 1-(3-(4-amidinophényl)-2-oxo-5-oxazolidinylméthyl)-pipéridine-4-carboxylique.

**3.** Procédé pour la préparation d'un composé de formule I selon la revendication 1, ainsi que de ses sels, caractérisé

(a) en ce que l'on libère un composé de formule I de l'un de ses dérivés fonctionnels par traitement avec un agent solvolysant ou hydrogénolysant ou
(b) en ce que l'on fait réagir un composé de formule II

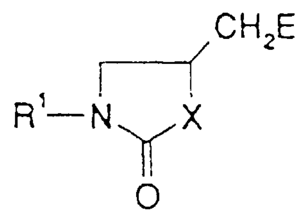

où

E représente Cl, Br, I ou un groupe OH estérifié réactif et
$R^1$ et X ont les significations indiquées dans la revendication 1,

avec un composé aminé de formule III

$$H\text{-}Y \qquad III$$

où

Y a la signification indiquée dans la revendication 1 ou

(c) en ce que l'on fait réagir un composé de formule IV

$$R\text{-}NH\text{-}CH_2\text{-}CH(XH)\text{-}CH_2\text{-}Y \qquad IV$$

où $R^1$, X et Y ont les significations indiquées dans la revendication 1 ou l'un de ses dérivés réactifs, avec un dérivé réactif de l'acide carbonique ou
(d) en ce que l'on traite, pour la préparation d'un composé guanidino de formule I ($R^1$ représente un reste phényle monosubstitué par $H_2N$-C(=NH)-NH-), un composé aminé correspondant à la formule I, mais contenant à la place du reste $R^1$ un groupe aminophényle, avec un agent amidinant,

et/ou en ce que l'on transforme dans un composé de formule I le ou les deux restes $R^1$ et/ou Y en un ou deux autres restes $R^1$ et/ou Y et/ou en ce que l'on transforme un composé de formule I en l'un de ses sels par traitement avec un acide ou une base.

4. Procédé pour la fabrication de préparations pharmaceutiques, caractérisé en ce que l'on met sous la forme d'un dosage approprié un composé de formule I selon la revendication 1 et/ou l'un de ses sels physiologiquement acceptables avec au moins un support ou un adjuvant solide, liquide ou semi-liquide.

5. Préparation pharmaceutique caractérisée en ce qu'elle contient au moins un composé de formule I selon la revendication 1 et/ou l'un de ses sels physiologiquement acceptables.

6. Utilisation des composés de formule I selon la revendication 1 ou de leurs sels physiologiquement acceptables pour la fabrication d'un médicament.

7. Utilisation des composés de formule I selon la revendication 1 ou de leurs sels physiologiquement acceptables pour lutter contre les thromboses, les infarctus du myocarde, l'apoplexie, l'ostéoporose, l'artériosclérose, les inflammations et/ou les tumeurs.